(19) Europäisches Patentamt / European Patent Office / Office européen des brevets

(11) **EP 3 801 223 B1**

(12) **EUROPÄISCHE PATENTSCHRIFT**

(45) Veröffentlichungstag und Bekanntmachung des Hinweises auf die Patenterteilung:
**25.05.2022 Patentblatt 2022/21**

(21) Anmeldenummer: **19729730.2**

(22) Anmeldetag: **06.06.2019**

(51) Internationale Patentklassifikation (IPC):
**A61B 5/02** (2006.01)

(52) Gemeinsame Patentklassifikation (CPC):
**A61B 5/02035; A61M 60/135; A61M 60/205; A61M 60/50;** A61M 60/148; A61M 2205/3334; A61M 2205/3344; A61M 2205/3365

(86) Internationale Anmeldenummer:
**PCT/EP2019/064808**

(87) Internationale Veröffentlichungsnummer:
**WO 2019/234167 (12.12.2019 Gazette 2019/50)**

(54) **BESTIMMVORRICHTUNG UND VERFAHREN ZUM BESTIMMEN EINER VISKOSITÄT EINES FLUIDS**

DETERMINATION APPLIANCE AND METHOD FOR DETERMINING A VISCOSITY OF A FLUID

DISPOSITIF DE DÉTERMINATION ET PROCÉDÉ POUR DÉTERMINER LA VISCOSITÉ D'UN FLUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.06.2018 DE 102018208936**

(43) Veröffentlichungstag der Anmeldung:
**14.04.2021 Patentblatt 2021/15**

(73) Patentinhaber: **Kardion GmbH**
**70376 Stuttgart (DE)**

(72) Erfinder:
- **STOTZ, Ingo**
  **71254 Ditzingen (DE)**
- **SCHLEBUSCH, Thomas, Alexander**
  **71272 Renningen (DE)**

(74) Vertreter: **Gauss, Nikolai et al**
**Pfiz/Gauss Patentanwälte PartmbB**
**Tübingerstraße 26**
**70178 Stuttgart (DE)**

(56) Entgegenhaltungen:
EP-A1- 3 088 016      WO-A1-92/15239
JP-A- H08 327 527

**Beschreibung**

Die Erfindung betrifft ein Herzunterstützungssystem und Verfahren zum Bestimmen einer Viskosität eines Fluids.

PT (Prothrombinzeit) und INR (International Normalized Ratio) sind das Standardmaß für die Blutgerinnung. Üblicherweise wird das INR in Blutproben durch Zugabe von Thromboplastin und anschließender Messung der Zeit bis zur Gerinnung bestimmt. Die Bestimmung kann im Labor erfolgen, es sind mittlerweile auch Teststreifengeräte zur Selbstmessung durch den Patienten verfügbar, vergleichbar mit dem Ablauf einer Blutzuckermessung. Für Patienten mit Herzunterstützungssystemen ist das sogenannte Gerinnungsmanagement zur Minimierung von Pumpenthrombosen essenziell. Möglicherweise ist für das Gerinnungsmanagement die Überwachung der Blutviskosität als INR-Ersatzparameter ausreichend.

Die EP 2 175 770 B1 beschreibt einen expliziten Blut-Viskositätssensor auf Basis von Oberflächenwellen, kurz SAW, zur Bestimmung der Viskosität.

Die US 7,591,777 B2 beschreibt eine Viskositätsbestimmung in Herzunterstützungssystemen durch die mechanische Rückwirkung der Blutviskosität auf den Antrieb des Herzunterstützungssystems.

Die EP 3 088 016 A1 offenbart eine VAD (ventricular assist device) Blutpumpe mit einem Rotor, mit einem mit dem Rotor gekoppelten Elektromotor, der für das Antreiben des Rotors mit einer Drehzahl dient, mit einer Drucksensoreinrichtung und mit einer Bestimmeinrichtung für das Bestimmen der Viskosität des Bluts in dem Körper des Patienten, mit einem in dem Körper eines Patienten implantierbaren Einlassstutzen mit einer Spitze, wobei der Einlassstutzen eine Aufnahmeschnittstelle hat, durch die das Blut des Patienten in das Innere des Einlassstutzens einströmen kann, und einer Auslasskanüle mit einer Auslassschnittstelle durch die das Blut aus dem Inneren der Kanüle austreten kann, wobei der Rotor zum Fördern des Bluts von der Aufnahmeschnittstelle zu der Auslassschnittstelle der Kanüle dient, wobei die Drucksensoreinrichtung für das Sensieren einer Druckdifferenz des Drucks des Bluts ausgebildet ist, und wobei die Bestimmeinrichtung dazu ausgebildet ist, die Viskosität des Bluts in dem Körper des Patienten unter Verwendung eines bestimmten Volumenstroms des Bluts und der sensierten Druckdifferenz des Bluts und der Drehzahl des Rotors zu bestimmen. Aufgabe der Erfindung ist es, ein verbessertes Verfahren zum Bestimmen einer Viskosität eines Fluids und ein verbessertes Herzunterstützungssystem hierzu anzugeben. Insbesondere ist es eine Aufgabe der Erfindung, ein Verfahren und ein Herzunterstützungssystem anzugeben, die das Bestimmen der Viskosität eines Fluids fortlaufend und auf einer kurzen Zeitskala ermöglicht.

Diese Aufgabe wird durch das in Anspruch 1 angegebene Herzunterstützungssystem und das in Anspruch 6 angegebene Verfahren gelöst. Vorteilhafte Ausführungsformen der Erfindung sind in den abhängigen Ansprüchen angegeben.

Nachfolgend wird eine als ein Herzunterstützungssystem einsetzbare Bestimmvorrichtung zum Bestimmen einer Viskosität eines Fluids und ein Verfahren zum Bestimmen einer Viskosität eines Fluids sowie schließlich ein entsprechendes Computerprogramm vorgestellt, die hilfreich für das Verständnis der vorliegenden Erfindung sind. Gegenstand der beanspruchten Erfindung sind jedoch nur ein Herzunterstützungssystem und ein Verfahren, die sämliche in den Ansprüchen 1 und 6 definierten Merkmale aufweisen.

Die mit dem vorgestellten Ansatz erreichbaren Vorteile bestehen darin, dass eine hier vorgestellte Bestimmvorrichtung dazu ausgebildet ist, um die Viskosität eines Fluids schnell und einfach unter Verwendung von aktuellen Strömungsparametern des Fluids zu bestimmen und bereitzustellen oder zu senden.

Es wird eine Bestimmvorrichtung zum Bestimmen einer Viskosität eines Fluids vorgestellt. Die Bestimmvorrichtung weist zumindest eine Bestimmeinrichtung und eine Bereitstellungseinrichtung auf. Die Bestimmeinrichtung ist dazu ausgebildet, um unter Verwendung zumindest eines sensierten Volumenstroms des Fluids und einer sensierten Druckdifferenz des Fluids und/oder einer Drehzahl eines Laufrads zum Fördern des Fluids die Viskosität des Fluids zu bestimmen. Die Bereitstellungseinrichtung ist dazu ausgebildet, um ein Viskositätssignal bereitzustellen oder zu senden, das die von der Bestimmeinrichtung bestimmte Viskosität repräsentiert.

Die Bestimmeinrichtung kann dazu ausgebildet sein, um die Viskosität unter Verwendung eines Funktionszusammenhangs zwischen dem Volumenstrom und der Druckdifferenz zu der Viskosität und/oder unter Verwendung einer Nachschlagetabelle zu bestimmen, insbesondere wobei in der Nachschlagetabelle ein Zusammenhang zwischen dem Volumenstrom und der Druckdifferenz zu der Viskosität abgelegt sein kann. So kann unter Verwendung des sensierten Volumenstroms und der sensierten Druckdifferenz schnell und einfach aus der Nachschlagetabelle eine diesen Werten zugeordnete Viskosität ausgelesen werden. Oder es kann unter Verwendung des sensierten Volumenstroms und der sensierten Druckdifferenz schnell und einfach durch Lösen des Funktionszusammenhangs die Viskosität bestimmt werden. Zum Erstellen der Nachschlagetabelle kann beispielsweise im Vorfeld eine Kalibrierung einer Messung der Art durchgeführt werden oder worden sein, dass sowohl die Viskosität im relevanten Bereich als auch eine Drehzahl beispielsweise einer Pumpeneinrichtung zum Fördern des Fluids im relevanten Bereich variieren und die resultierenden Pumpenflüsse gemessen werden oder wurden. Alternativ oder zusätzlich kann auf Basis der Kalibrierungswerte eine beispielsweise empirische Funktion bestimmt werden oder worden sein, mit deren Hilfe sich die Viskosität anschließend berechnen lässt. Die Nachschlagetabelle und/oder der Funktionszusammenhang kann in der Bestimmeinrichtung gespeichert sein oder von der Bestimmeinrichtung zur Verwendung einlesbar sein.

Die Bestimmeinrichtung kann beispielsweise auch außerhalb eines Körpers eines Patienten angeordnet sein und Daten wie des vorstehend genannten sensierten Volumenstroms des Fluids und einer sensierten Druckdifferenz des Fluids und/oder einer Drehzahl einer Pumpe die Viskosität des Fluids zu bestimmen. Hierzu kann dann beispielsweise die Bestimmeinrichtung die zur Bestimmung der Viskosität erforderlichen Werte bzw. Parameter drahtlos oder über eine Signalleitung übermittelt erhalten, sodass sie die Viskosität des Fluids auch außerhalb des Körpers des Patienten bestimmen kann.

Die Bestimmvorrichtung kann eine Kanüle mit einer Aufnahmeschnittstelle zur Aufnahme des Fluids und einer der Aufnahmeschnittstelle gegenüberliegenden Auslassschnittstelle zum Auslassen des Fluids aufweisen, insbesondere wobei die Druckdifferenz eine Differenz zwischen einem Druck des Fluids im Bereich der Aufnahmeschnittstelle und einem weiteren Druck des Fluids im Bereich der Auslassschnittstelle und/oder der Volumenstrom einen Volumenstrom des Fluids durch die Kanüle repräsentieren kann. Eine derartige Kanüle kann zur Verwendung an oder in dem Herzunterstützungssystem ausgeformt sein. Beispielsweise kann die Kanüle dazu ausgeformt oder ausgebildet sein, um Blut als das Fluid aufzunehmen. So kann vorteilhafterweise unter Verwendung der Bestimmvorrichtung die aktuelle Viskosität des Bluts in der Kanüle bestimmt werden.

Die Bestimmvorrichtung weist ein Laufrad zum Fördern des Fluids von der Aufnahmeschnittstelle zu der Auslassschnittstelle der Kanüle auf, insbesondere wobei das Laufrad an oder im Bereich der Auslassschnittstelle angeordnet oder anordenbar ist. Das Laufrad kann beispielsweise in einem Auslassabschnitt benachbart zu der Auslassschnittstelle angeordnet sein. Im Betrieb des Laufrads können so der Volumenstrom des Fluids und die Druckdifferenz bewirkt werden.

Hierbei ist es von Vorteil, wenn die Bestimmvorrichtung einen Volumenstromsensor aufweist, der dazu ausgebildet ist, um einen Volumenstrom des Fluids durch die Kanüle zu sensieren und an die Bestimmeinrichtung bereitzustellen oder zu senden, insbesondere wobei der Volumenstromsensor im Bereich der Aufnahmeschnittstelle angeordnet ist. So kann zum Bestimmen der Viskosität ein aktueller Volumenstrom berücksichtigt werden.

Dieser Volumenstromsensor kann zumindest einen Doppler-Sensor zum Sensieren eines Doppler-Ultraschalls und/oder einen Thermofilament-Anemometrie-Sensor und/oder einen optischen Sensor aufweisen. Der Thermofilament-Anemometrie-Sensor kann ein Sensorelement, beispielsweise einen Draht, aufweisen, wobei das Sensorelement elektrisch beheizt werden kann und dessen elektrischer Widerstand von der Temperatur abhängt. Durch die Umströmung kann ein Wärmetransport in das Fluid stattfinden, der sich mit der Strömungsgeschwindigkeit verändert. Durch Messung der elektrischen Größen kann so auf die Strömungsgeschwindigkeit geschlossen werden.

Die Bestimmvorrichtung kann außerdem eine Drucksensoreinrichtung mit zumindest einem differenziellen Drucksensor und/oder zwei barometrischen Drucksensoren aufweisen, insbesondere wobei die Drucksensoreinrichtung dazu ausgebildet sein kann, um eine Druckdifferenz zwischen zwei Sensorpunkten auf zwei gegenüberliegenden Seiten des Laufrads zu sensieren und an die Bestimmeinrichtung bereitzustellen oder zu senden. So kann zum Bestimmen der Viskosität eine aktuelle Druckdifferenz berücksichtigt werden.

Die Bestimmvorrichtung weist eine mit dem Laufrad gekoppelte oder koppelbare Antriebseinrichtung zum Antreiben des Laufrads auf, insbesondere wobei die Bestimmeinrichtung dazu ausgebildet sein kann, um die Viskosität unter Verwendung eines Antriebsparameters der Antriebseinrichtung und/oder des Laufrads zu bestimmen. Hierbei kann die Bestimmeinrichtung dazu ausgebildet sein, um die Viskosität unter Verwendung eines Antriebsparameters der Antriebseinrichtung und/oder des Laufrads während eines Betriebs der Antriebseinrichtung und/oder des Laufrads zu bestimmen. Als der Antriebsparameter kann eine elektrische Leistungsaufnahme der Antriebseinrichtung und/oder eine Drehzahl und/oder eine Winkelgeschwindigkeit des Laufrads verstanden werden. Eine derartige Bestimmvorrichtung kann als ein Herzunterstützungssystem ausgeformt oder einsetzbar sein. Dieses Herzunterstützungssystem kann vorteilhafterweise eine aktuelle Blutviskosität bestimmen und beispielsweise für ein Diagnoseverfahren bereitstellen oder senden.

Weiterhin wird ein Verfahren zum Bestimmen einer Viskosität eines Fluids vorgestellt. Das Verfahren weist einen Schritt des Bestimmens und einen Schritt des Bereitstellens auf. Im Schritt des Bestimmens wird die Viskosität des Fluids unter Verwendung zumindest eines sensierten Volumenstroms des Fluids und einer sensierten Druckdifferenz des Fluids bestimmt. Im Schritt des Bereitstellens wird ein Viskositätssignal bereitgestellt oder gesendet, das die im Schritt des Bestimmens bestimmte Viskosität repräsentiert.

Dieses Verfahren kann unter Verwendung der vorangehend vorgestellten Bestimmvorrichtung durchführbar sein. Das Verfahren kann beispielsweise in Software oder Hardware oder in einer Mischform aus Software und Hardware beispielsweise in einem Steuergerät implementiert sein.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens verwendet wird, insbesondere wenn das Programmprodukt oder Programm auf einem Computer oder einer Vorrichtung ausgeführt wird.

Ausführungsbeispiele des hier vorgestellten Ansatzes sind in den Zeichnungen dargestellt und in der nachfolgenden Beschreibung näher erläutert. Es zeigt:

Fig. 1    eine schematische Darstellung einer Bestimmvorrichtung zum Bestimmen einer Viskosität eines Fluids gemäß einem Ausführungsbeispiel;

Fig. 2    eine schematische Seitenansicht einer Bestimmvorrichtung gemäß einem Ausführungsbeispiel;

Fig. 3    ein Kennfeld aus Druckdifferenz über Volumenstrom für verschiedene Viskositäten zur Verwendung mit einer Bestimmvorrichtung gemäß einem Ausführungsbeispiel; und

Fig. 4    ein Ablaufdiagramm eines Verfahrens zum Bestimmen einer Viskosität eines Fluids gemäß einem Ausführungsbeispiel.

In der nachfolgenden Beschreibung günstiger Ausführungsbeispiele des vorliegenden Ansatzes werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

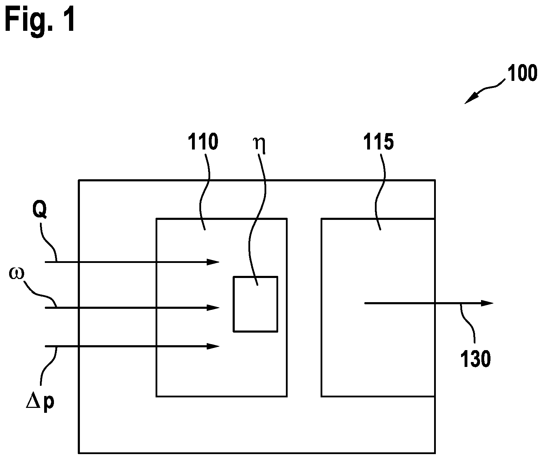

Fig. 1 zeigt eine schematische Darstellung einer Bestimmvorrichtung 100 zum Bestimmen einer Viskosität $\eta$ eines Fluids gemäß einem Ausführungsbeispiel.

Die Bestimmvorrichtung 100 weist eine Bestimmeinrichtung 110 und eine Bereitstellungseinrichtung 115 auf. Die Bestimmeinrichtung 110 ist dazu ausgebildet, um unter Verwendung zumindest eines sensierten Volumenstroms Q des Fluids und einer sensierten Druckdifferenz $\Delta p$ des Fluids die Viskosität $\eta$ des Fluids zu bestimmen. Die Bereitstellungseinrichtung 115 ist dazu ausgebildet, um ein Viskositätssignal 130 bereitzustellen oder zu senden, das die von der Bestimmeinrichtung 110 bestimmte Viskosität $\eta$ repräsentiert. Gemäß diesem Ausführungsbeispiel ist die Bestimmeinrichtung 110 dazu ausgebildet, um den sensierten Volumenstrom Q und die sensierte Druckdifferenz $\Delta p$ in Form von Sensorsignalen einzulesen.

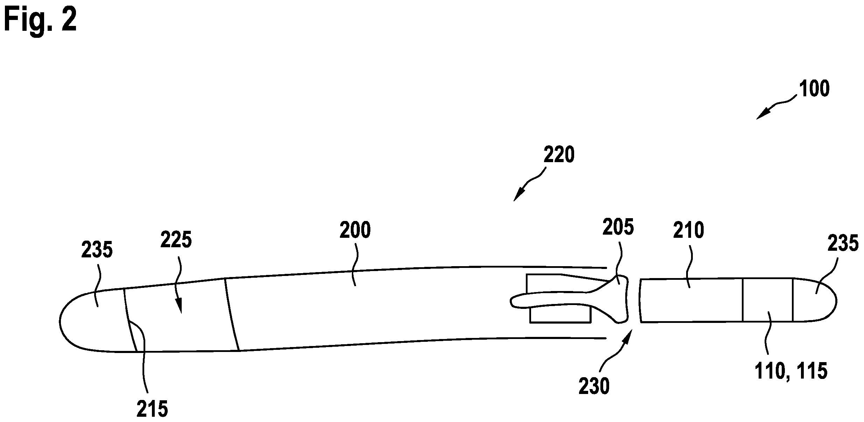

Fig. 2 zeigt eine schematische Seitenansicht einer Bestimmvorrichtung 100 gemäß einem Ausführungsbeispiel der Erfindung.

Dabei kann es sich um die anhand von Figur 1 beschriebene Bestimmvorrichtung 100 handeln, mit dem Unterschied, dass die Bestimmvorrichtung 100 gemäß diesem Ausführungsbeispiel zusätzlich eine Kanüle 200, ein Laufrad 205, eine Antriebseinrichtung 210, einen Volumenstromsensor 215 und eine Drucksensoreinrichtung 220 aufweist.

Die Bestimmvorrichtung oder eine Bestimmeinrichtung 110 kann in der Pumpe integriert werden oder außerhalb eines Körpders eines Patienten angeordnet werden, wenn für mikroelektronische Elemente zur Messung von Parametern, die zur Bestimmung der Viskosität erforderlich sind, kein oder lediglich ein zu geringer Bauraum zur Verfügung steht. In diesem Fall kann bespielsweise die Elektronik bzw. entsprechende Komponenten der Bestimmeinrichtung 110 in einem abgesetzten (implantierten) Steuergerät untergebracht werden, sodass dann vor allem ein Drucksensor und/oder ein Wandlerelement des Volumenstromsensors in der Pumpe selbst untergebracht werden bzw. im Patienten implantiert werden können. Die Sensorwerte von dem oder den entsprechenden, im Patienten implantierten Sensor(en) können dann beispielsweise drahtlos oder mittels einer Signalleitung aus dem Patienten herausgeleitet werden und in der Bestimmeinrichtung 110, beispielsweise in einer Tasche oder an einem Gürtel des Patienten verarbeitet werden, um die Viskosität des Fluids (hier des Bluts) zu bestimmen. Ein solches Ausführungsbeispiel ist in den hier beigefügten Figuren nicht explizit dargestellt.

Zusätzlich oder alternativ kann natürlich auch eine Bestimmung der Viskosität des Fluids durch eine Bestimmeinrichtung 110 in der Form eines Cloud-Servers erfolgen, sodass in diesem Fall eine Übertragung der für die Bestimmung erforderlichen Sensorwerte über das Internet bzw. eine entsprechende Signalleitung durchzuführen ist. Eine entsprechende Sicherung bzw. Verschlüsselung dieser Daten gegen einen unbefugten Abgriff bzw. ein Auslesen dieser Daten durch unbefugte Personen sollte hierbei vorteilhafterweise sichergestellt werden.

Denkbar ist somit eine Anrodnung der Bestimmeinrichtung 110 in drei Optionen:

1)    Berechnung der Viskosität durch eine in den Patienten implantierte Bestimmeinrichtung 110 (z. B als Steuergerät oder auch in einer Pumpe (speziell, wenn entsprechende Hardware-Komponenten ausreichend geringe Dimensionen aufweisen)

2)    Berechnung der Viskosität extrakorporal beispielsweise körpernah am Patienten (z. B. in einem Kästchen, das an einem Gürtel des Patienten befestigt ist)

3) Berechnung der Viskosität weiter abgesetzt vom Patienten (beispielsweise in einer nicht-tragbaren Komponente, wie einem Tischgerät, einem Analysegerät in einer Arztpraxis oder gar einem Cloudserver.

Die Kanüle 200 weist eine Aufnahmeschnittstelle 225, die zur Aufnahme des Fluids ausgeformt ist, und eine der Aufnahmeschnittstelle 225 gegenüberliegende Auslassschnittstelle 230, die zum Auslassen des Fluids ausgeformt ist, auf.

Das Laufrad 205 ist dazu ausgebildet, um das Fluid von der Aufnahmeschnittstelle 225 zu der Auslassschnittstelle 230 der Kanüle 200 zu fördern. Gemäß diesem Ausführungsbeispiel ist das Laufrad 205 im Bereich der Auslassschnittstelle 230 und/oder in der Kanüle 200 angeordnet.

Der Volumenstromsensor 215 ist dazu ausgebildet, um einen Volumenstrom des Fluids durch die Kanüle 200 zu sensieren und an die Bestimmeinrichtung 110 bereitzustellen oder zu senden. Demnach repräsentiert der Volumenstrom einen Volumenstrom des Fluids durch die Kanüle 200. Gemäß diesem Ausführungsbeispiel ist der Volumenstromsensor 215 hierzu im Bereich der Aufnahmeschnittstelle 225 angeordnet. Der Volumenstromsensor 215 weist gemäß diesem Ausführungsbeispiel einen Doppler-Ultraschallsensor auf. Gemäß einem alternativen Ausführungsbeispiel weist der Volumenstromsensor 215 zusätzlich einen Thermofilament-Anemometrie-Sensor und/oder einen optischen Sensor auf. In einem nicht zur beanspruchten Erfindung gehörenden Besipiel weist der Volumenstromsensor alternativ zu einem Doppler-Ultraschallsensor einen Thermofilament-Anemometrie-Sensor und/oder einen optischen Sensor auf.

Die Drucksensoreinrichtung 220 weist gemäß diesem Ausführungsbeispiel zwei barometrische Drucksensoren 235 auf, die dazu ausgebildet sind, um eine Druckdifferenz zwischen zwei Sensorpunkten auf zwei gegenüberliegenden Seiten des Laufrads 205 zu sensieren und an die Bestimmeinrichtung 110 bereitzustellen oder zu senden. Gemäß einem alternativen Ausführungsbeispiel weist die Drucksensoreinrichtung 220 zusätzlich oder alternativ zumindest einen differenziellen Drucksensor auf. Die Drucksensoren 235 sind gemäß diesem Ausführungsbeispiel im Bereich der Aufnahmeschnittstelle 225 und im Bereich der Auslassschnittstelle 230 angeordnet. Demnach repräsentiert die Druckdifferenz gemäß diesem Ausführungsbeispiel eine Differenz zwischen einem Druck des Fluids im Bereich der Aufnahmeschnittstelle 225 und einem weiteren Druck des Fluids im Bereich der Auslassschnittstelle 230. Die Antriebseinrichtung 210 ist mit dem Laufrad 205 gekoppelt und dazu ausgebildet, um das Laufrad 205 anzutreiben. Gemäß diesem Ausführungsbeispiel ist die Bestimmeinrichtung 110 dazu ausgebildet, um die Viskosität unter Verwendung eines Antriebsparameters der Antriebseinrichtung 210 und/oder des Laufrads 205 während eines Betriebs der Antriebseinrichtung 210 und/oder des Laufrads 205 zu bestimmen.

Hierbei kann beispielsweise die Bestimmeinrichtung 110 außerhalb des Patienten bzw. einer Pumpe z. B. in einem portablen Steuergerät angeordnet sein. Sensorwerte von im Patienten implantierten Sensoren können dann beispielsweise drahtlos bzw. mittels einer Signalleitung der Bestimmeinrichtung 110 zugeführt werden.

Im Folgenden werden Details der Bestimmvorrichtung 100 noch einmal mit anderen Worten genauer beschrieben:

Die hier vorgestellte Bestimmvorrichtung 100 ist gemäß diesem Ausführungsbeispiel als ein Herzunterstützungssystem einsetzbar. Für Patienten mit einem Herzunterstützungssystem, auch VAD-Patienten genannt, VAD steht für "Ventricular Assist Device", ist das Gerinnungsmanagement zur Minimierung von Pumpenthrombosen essenziell. Die Patienten werden dazu beispielsweise mit Arzneimitteln zur Hemmung der plasmatischen Blutgerinnung therapiert und der INR wird so beispielsweise im Bereich 2 bis 2,5 eingestellt.

Eine mechanische Last auf die Antriebseinrichtung 210 eines VAD-Systems, also eines Herzunterstützungssystems, ist vom Volumenstrom, der Druckdifferenz und der Viskosität abhängig. Bei bekanntem Volumenstrom und bekannter Druckdifferenz, welche bei der hier vorgestellten Bestimmvorrichtung 100 über Sensoren 215, 220 gemessen werden, kann aus der elektrischen Leistungsaufnahme der Antriebseinrichtung 210 auf die Viskosität des Fluids, hier Blut, geschlossen werden. Hierzu ist die Bestimmeinrichtung 110 gemäß diesem Ausführungsbeispiel dazu ausgebildet, um als den Antriebsparameter einen Parameter einzulesen, der die mechanische Last auf die Antriebseinrichtung 210 und/oder das Laufrad 205 repräsentiert oder bestimmbar macht. Hierbei ist die Bestimmeinrichtung 110 vorteilhafterweise dazu ausgebildet, um die Leistungsaufnahme der Pumpe, bestehend aus Antriebseinrichtung 210 und Laufrad 205, auf einen Volumenstrombeitrag und einen Viskositätsbeitrag aufzuteilen. Die Flussmessung wird gemäß diesem Ausführungsbeispiel ultraschallbasiert oder gemäß einem alternativen Ausführungsbeispiel anemometrisch realisiert. Vorteilhafterweise ist hierbei durch eine explizite Doppler-Ultraschall-Volumenstrommessung eine direkte Bestimmung der Viskosität während des Betriebs der Bestimmvorrichtung 100 möglich. Eine Pumpleistung der Pumpe muss dafür vorteilhafterweise nicht unterbrochen werden.

Die Blutviskosität wird gemäß diesem Ausführungsbeispiel im Betrieb der Bestimmvorrichtung 100 von der Bestimmeinrichtung 110 kontinuierlich oder gemäß einem alternativen Ausführungsbeispiel in festen Zeitintervallen erhoben. Die Bereitstellungseinrichtung 115 ist dazu ausgebildet, um die bestimmte Viskosität einem Arzt und/oder Patienten als einen Parameter zur Therapieführung bereitzustellen. Hierzu ist das Viskositätssignal dazu ausgebildet, um die Viskosität auf einem Display anzuzeigen und/oder durch Funkübertragung in einen Webdienst zu übermitteln. Wie bereits zuvor ausgeführt, kann auch die Bestimmeinrichtung 110 außerhalb des Patienten angeordnet werden, beispielsweise in einer Tasche, die der Patient mitführt. Signalwerte von im Patienten implantierten Sensoren können dann beispielsweise drahtlos und/oder mittels einer Signalleitung an die Bestimmvorrichtung übertragen werden.

Eine hier vorgestellte Bestimmvorrichtung 100 beinhaltet ein System bestehend aus einem Pumpenantrieb in Form der Antriebseinrichtung 210, dem Laufrad 205 und der Kanüle 200, auch Zulaufkanüle genannt, dem Volumenstromsensor 215 zur Messung des tatsächlichen von Antrieb und Laufrad 205 geförderten Pumpenvolumenstroms, hier über Doppler-Ultraschall, optional oder zusätzlich über Thermofilament-Anemometrie und/oder optische Verfahren. Hier dargestellt ist eine Integration des Volumenstromsensors 215 gemäß der beanspruchten Erfindung in Form eines Doppler-Ultraschallsensors in einer Spitze der Zulaufkanüle. Zudem umfasst die Bestimmvorrichtung 100 zwei barometrische Drucksensoren 235 zur Bildung der Druckdifferenz in der Bestimmeinrichtung 110, die gemäß diesem Ausführungsbeispiel ein Datenverarbeitungsgerät in Form eines Mikrocontrollers aufweist. Gemäß einem alternativen Ausführungsbeispiel weist die Bestimmvorrichtung 100 zumindest einen differenziellen Drucksensor zur Bestimmung eines Druckgefälles über dem Laufrad 205 in Form eines Impellers auf.

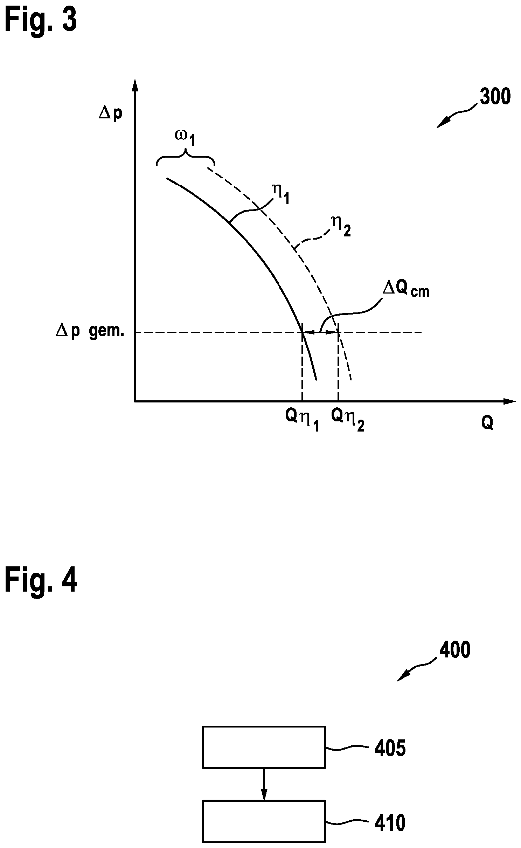

Es folgen Rechenbeispiele zur Veranschaulichung möglicher Methoden der Bestimmeinrichtung 110 beim Bestimmen der Viskosität, siehe hierzu auch Figur 3:

Die hydraulische Leistung der Pumpe $P_{hydraulisch}$ ist von einer Winkelgeschwindigkeit $\omega$, einem hydraulischen Wirkungsgrad $\eta_{hydraulisch}$ und einem Lastmoment M abhängig, wobei das Lastmoment M von der Viskosität abhängig ist. Dieses Verhältnis lässt sich in folgender Gleichung darstellen:

$$P_{hydraulisch} = \omega \cdot M \cdot \eta_{hydraulisch}$$

Die hydraulische Leistung $P_{hydraulisch}$ ist ebenfalls von der Druckdifferenz $\Delta p$ und dem Volumenstrom Q oder Volumenfluss abhängig. Dieses Verhältnis lässt sich in folgender Gleichung darstellen:

$$P_{hydraulisch} = \Delta p \cdot Q$$

Wird die Pumpe nun bei einer definierten Winkelgeschwindigkeit $\omega_1$ betrieben und der tatsächliche Volumenstrom $Q_{\omega 1}$ gemessen, so kann aus diesem gemessenen Volumenstrom $Q_{\omega 1}$, gemäß diesem Ausführungsbeispiel mittels Doppler-Ultraschallsensorik, die Viskosität $\eta$ bestimmt werden, wie in Fig. 3 illustriert. Hierzu ist gemäß diesem Ausführungsbeispiel im Vorfeld eine Kalibrierung der Messung der Art durchgeführt worden, dass sowohl die Viskosität im relevanten Bereich als auch die Drehzahl im relevanten Bereich variierten und die resultierenden Pumpenflüsse gemessenen wurden. Hieraus wurden dann Nachschlagetabellen, sogenannte "Lookup-Tabellen", kurz LUT, erstellt, mit deren Hilfe dann von der Bestimmeinrichtung 110 einer gemessenen Druckdifferenz und einem gemessenen Volumenstrom bei gegebener Drehzahl/Winkelgeschwindigkeit eine Viskosität zuordenbar ist. Diese Messung und Kalibrierung wurde gemäß diesem Ausführungsbeispiel unter Verwendung der Bestimmvorrichtung 100 durchgeführt. Gemäß einem alternativen Ausführungsbeispiel wird die Viskosität von der Bestimmeinrichtung 110 ermittelt, indem auf Basis der Kalibrierungswerte ein Funktionszusammenhang in Form einer empirischen Funktion bestimmt wird, mit deren Hilfe sich die Viskosität anschließend berechnen lässt:

$$\eta = f(\Delta p, Q, \omega)$$

Alternativ zur Verwendung der Winkelgeschwindigkeit w wird von der Bestimmeinrichtung 110 gemäß einem alternativen Ausführungsbeispiel die elektrische Leistungsaufnahme zur Berechnung herangezogen, da:

$$P_{el} = P_{hyd} / (\eta_{el} \cdot \eta_{mech} \cdot \eta_{hyd})$$

Diese ist hierbei in Vorversuchen über Messung des Moments und der Drehzahl sowie von Spannung U, und Strom I unter Verwendung der Bestimmvorrichtung 100 bestimmt worden. Unter der Prämisse, dass die weiteren mechanischen Verluste nur drehzahl- und druckabhängig sind, was bei einer der hier vorgestellten Bestimmvorrichtung 100 in sehr guter Näherung zutrifft, ist davon auszugehen, dass $\eta_{mech}$ konstant ist und dadurch keine Rolle für die Viskositätsermittlung spielt.

Der Volumenstromsensor 215 ist optisch oder per Durchleuchtung nachweisbar. Die Berechnung der Viskosität aus Druckdifferenz, Volumenstrom und/oder Winkelgeschwindigkeit lässt sich im gezielten Experiment durch Manipulation des Volumenstroms oder der Druckdifferenz nachweisen.

Fig. 3 zeigt ein Kennfeld 300 aus Druckdifferenz $\Delta p$ über Volumenstrom Q für verschiedene Viskositäten $\eta$ zur

Verwendung mit einer Bestimmvorrichtung gemäß einem Ausführungsbeispiel. Dabei kann es sich um eine der anhand der in den Figuren 1 oder 2 beschriebenen Bestimmvorrichtungen 100 handeln. Das Kennfeld 300 ist gemäß diesem Ausführungsbeispiel in Form der in Figur 2 beschriebenen Nachschlagetabelle oder des Funktionszusammenhangs in der Bestimmeinrichtung der Bestimmvorrichtung gespeichert oder von der Bestimmeinrichtung einlesbar.

$$\text{Für} \quad \Delta p_{gem} = \notin \quad \& \quad \omega = \notin$$

folgt

$$Q = f(\eta) \quad \text{mit} \quad Q_{\eta 2} > Q_{\eta 1} \quad \text{für} \quad \eta_1 > \eta_2.$$

Fig. 4 zeigt ein Ablaufdiagramm eines Verfahrens 400 zum Bestimmen einer Viskosität eines Fluids gemäß einem Ausführungsbeispiel. Dabei kann es sich um ein Verfahren 400 handeln, das von einer der anhand einer der Figuren 1 oder 2 beschriebenen Bestimmvorrichtungen ausführbar oder ansteuerbar ist.

Das Verfahren 400 weist einen Schritt 405 des Bestimmens und einen Schritt 410 des Bereitstellens auf. Im Schritt 405 des Bestimmens wird die Viskosität des Fluids unter Verwendung zumindest eines sensierten Volumenstroms des Fluids und einer sensierten Druckdifferenz des Fluids und/oder einer Drehzahl eines Laufrads zum Fördern des Fluids bestimmt. Im Schritt 410 des Bereitstellens wird ein Viskositätssignal bereitgestellt oder gesendet, das die im Schritt 405 des Bestimmens bestimmte Viskosität repräsentiert.

Die hier vorgestellten Verfahrensschritte können wiederholt sowie in einer anderen als in der beschriebenen Reihenfolge ausgeführt werden.

**Patentansprüche**

1.  Herzunterstützungssystem mit einem Laufrad, mit einer mit dem Laufrad (205) gekoppelten Antriebseinrichtung (210), die für das Antreiben des Laufrads (205) mit einer Drehzahl (w) dient, mit einer Drucksensoreinrichtung (220) und mit einer Bestimmeinrichtung (110) für das Bestimmen der Viskosität ($\eta$) des Bluts in dem Körper des Patienten, mit einer in dem Körper eines Patienten implantierbaren Kanüle (200) mit einer Spitze, wobei die Kanüle (200) eine Aufnahmeschnittstelle (225) hat, durch die das Blut des Patienten in das Innere der Kanüle (200) einströmen kann, und eine Auslassschnittstelle (230) aufweist, die der Aufnahmeschnittstelle (225) gegenüber liegt, durch die das Blut aus dem Inneren der Kanüle (200) austreten kann, wobei das Laufrad (205) zum Fördern des Bluts von der Aufnahmeschnittstelle (225) zu der Auslassschnittstelle (230) der Kanüle (200) dient, einen in der Spitze der Kanüle (200) angeordneten, als ein Doppler-Ultraschallsensor ausgebildeter Volumenstromsensor (215) für das Sensieren eines Volumenstroms (Q) des Bluts des Patienten durch die Kanüle (200), wobei die Drucksensoreinrichtung (220) für das Sensieren einer Druckdifferenz ($\Delta p$) des Drucks des Bluts in dem Bereich der Aufnahmeschnittstelle (225) und des Drucks des Bluts in dem Bereich der Auslassschnittstelle (230) ausgebildet ist, und wobei die Bestimmeinrichtung (110) dazu ausgebildet ist, die Viskosität ($\eta$) des Bluts in dem Körper des Patienten unter Verwendung zumindest des sensierten Volumenstroms (Q) des Bluts und der sensierten Druckdifferenz ($\Delta p$) des Bluts und der Drehzahl ($\omega$) des Laufrads (205) oder alternativ hierzuzu unter Verwendung zumindest des sensierten Volumenstroms (Q) des Bluts und der sensierten Druckdifferenz ($\Delta p$) des Bluts und einer elektrischen Leistungsaufnahme Pei der Antriebseinrichtung (210) für das Laufrad (205) zu bestimmen.

2.  Herzunterstützungssystem nach Anspruch 1, **dadurch gekennzeichnet, dass** die Bestimmeinrichtung (110) dazu ausgebildet ist, die Viskosität ($\eta$) unter Verwendung eines Funktionszusammenhangs zwischen dem Volumenstrom (Q) und der Druckdifferenz ($\Delta p$) zu der Viskosität ($\eta$) und/oder unter Verwendung einer Nachschlagetabelle zu bestimmen, insbesondere wobei in der Nachschlagetabelle ein Zusammenhang zwischen dem Volumenstrom (Q) und der Druckdifferenz ($\Delta p$) zu der Viskosität ($\eta$) abgelegt ist.

3.  Herzunterstützungssystem nach Anspruch 1 oder 2, **dadurch gekennzeichnet, dass** das Laufrad (205) an oder im Bereich der Auslassschnittstelle (230) angeordnet ist.

4.  Herzunterstützungssystem (100) nach einem der Ansprüche 1 bis 3, **dadurch gekennzeichnet, dass** die Drucksensoreinrichtung (220) zumindest einen differenziellen Drucksensor und/oder zwei barometrischen Drucksensoren (235) aufweist.

5. Herzunterstützungssystem nach einem der Ansprüche 1 bis 4, **dadurch gekennzeichnet, dass** die Drucksensoreinrichtung (220) dazu ausgebildet ist, eine Druckdifferenz ($\Delta$p) zwischen zwei Sensorpunkten zu sensieren und an die Bestimmeinrichtung (110) bereitzustellen oder zu senden.

6. Verfahren (400) zum Bestimmen einer Viskosität ($\eta$) des Bluts eines Patienten in einem Herzunterstützungssystem mit einem Laufrad (205), mit einer mit dem Laufrad (205) gekoppelten Antriebseinrichtung (210), die für das Antreiben des Laufrads (205) mit einer Drehzahl (w) dient, mit einer in dem Körper eines Patienten implantierbaren Kanüle (200), die eine Aufnahmeschnittstelle (225) hat, durch die das Blut des Patienten in das Innere der Kanüle (200) einströmen kann, und die eine Auslassschnittstelle (230) aufweist, die der Aufnahmeschnittstelle (225) gegenüber liegt, durch die das Blut aus dem Inneren der Kanüle (200) austreten kann, wobei das Laufrad (205) zum Fördern des Bluts von der Aufnahmeschnittstelle (225) zu der Auslassschnittstelle (230) der Kanüle (200) dient,
bei dem die Viskosität ($\eta$) des Bluts unter Verwendung zumindest eines mittels eines in einer Spitze der Kanüle 200) angeordneten, als ein - Doppler-Ultraschallsensor ausgebildeten Volumenstromsensor (215) sensierten Volumenstroms (Q) des Bluts und einer sensierten Druckdifferenz ($\Delta$p) des Drucks des Bluts in dem Bereich der Aufnahmeschnittstelle (225) und des Drucks des Bluts in dem Bereich der Auslassschnittstelle (230) und der Drehzahl (w) des Laufrads (205) zum Fördern des Fluids bestimmt wird; oder, alternativ hierzu unter Verwendung zumindest eines mittels eines in einer Spitze der Kanüle angeordneten, als ein -Doppler-Ultraschallsensor ausgebildeten Volumenstromsensor (215) in dem Bereich der Aufnahmeschnittstelle (225) in der Kanüle (200) sensierten Volumenstroms (Q) des Bluts und einer sensierten Druckdifferenz ($\Delta$p) des Bluts in dem Bereich der Aufnahmeschnittstelle (225) und dem Bereich der Auslassschnittstelle (230) und einer elektrischen Leistungsaufnahme $P_{el}$ der Antriebseinrichtung (210) für das Laufrad (205) bestimmt wird.

## Claims

1. Ventricular assist system comprising an impeller, a drive device (210) which is coupled to the impeller (205) and is used to drive the impeller (205) at a speed ($\omega$), a pressure sensor device (220), and a determination device (110) for determining the viscosity ($\eta$) of the blood in the body of a patient, said determination device comprising a cannula (200) which can be implanted in the body of the patient and has a tip, wherein the cannula (200) has a receiving interface (225) through which the blood of the patient can flow into the interior of the cannula (200), and has an outlet interface (230) which is opposite the receiving interface (225) and through which the blood can exit from the interior of the cannula (200), wherein the impeller (205) is used to convey the blood from the receiving interface (225) to the outlet interface (230) of the cannula (200), and said determination device comprising a volume flow sensor (215) for sensing a volume flow (Q) of the blood of the patient through the cannula (200), which sensor is arranged in the tip of the cannula (200) and is designed as a Doppler ultrasonic sensor, wherein the pressure sensor device (220) is designed for sensing a pressure difference ($\Delta$p) of the pressure of the blood in the region of the receiving interface (225) and the pressure of the blood in the region of the outlet interface (230), and wherein the determination device (110) is designed to determine the viscosity ($\eta$) of the blood in the body of the patient using at least the sensed volume flow (Q) of the blood and the sensed pressure difference ($\Delta$P) of the blood and the speed ($\omega$) of the impeller (205) or alternatively using at least the sensed volume flow (Q) of the blood and the sensed pressure difference ($\Delta$p) of the blood and an electrical power input $P_{el}$ of the drive device (210) for the impeller (205).

2. Ventricular assist system according to claim 1, **characterized in that** the determination device (110) is designed to determine the viscosity ($\eta$) using a functional relationship between the volume flow (Q) and the pressure difference ($\Delta$p) with respect to the viscosity ($\eta$) and/or using a lookup table, in particular a relationship between the volume flow (Q) and the pressure difference ($\Delta$p) with respect to the viscosity ($\eta$) being stored in the lookup table.

3. Ventricular assist system according to either claim 1 or claim 2, **characterized in that** the impeller (205) is arranged on or in the region of the outlet interface (230).

4. Ventricular assist system (100) according to any of claims 1 to 3, **characterized in that** the pressure sensor device (220) has at least one differential pressure sensor and/or two barometric pressure sensors (235).

5. Ventricular assist system according to any of claims 1 to 4, **characterized in that** the pressure sensor device (220) is designed to sense a pressure difference ($\Delta$p) between two sensor points and to provide or transmit said pressure difference to the determination device (110).

6. Method (400) for determining a viscosity ($\eta$) of the blood of a patient in a ventricular assist system comprising an impeller (205), a drive device (210) which is coupled to the impeller (205) and is used to drive the impeller (205) at a speed ($\omega$), a cannula (200) which can be implanted in the body of a patient, has a receiving interface (225) through which the blood of the patient can flow into the interior of the cannula (200), and has an outlet interface (230) which is opposite the receiving interface (225) and through which the blood can exit from the interior of the cannula (200), wherein the impeller (205) is used to convey the blood from the receiving interface (225) to the outlet interface (230) of the cannula (200), in which method the viscosity ($\eta$) of the blood is determined using at least volume flow (Q) of the blood, which flow is sensed by means of a volume flow sensor (215) that is arranged in a tip of the cannula (200) and is designed as a Doppler ultrasonic sensor, and a sensed pressure difference ($\Delta$p) of the pressure of the blood in the region of the receiving interface (225) and the pressure of the blood in the region of the outlet interface (230) and the speed ($\omega$) of the impeller (205) for conveying the fluid; or alternatively is determined using at least one volume flow (Q) of the blood, which flow is sensed in the cannula (200) in the region of the receiving interface (225) by means of a volume flow sensor (215) that is arranged in a tip of the cannula and is designed as a Doppler ultrasonic sensor, and a sensed pressure difference ($\Delta$p) of the blood in the region of the receiving interface (225) and the region of the outlet interface (230), and an electrical power input $P_{el}$ of the drive device (210) for the impeller (205).

**Revendications**

1. Système d'assistance cardiaque comportant une roue à aubes, comportant un dispositif d'entraînement (210) accouplé à la roue à aubes (205) et servant à entraîner la roue à aubes (205) à une vitesse de rotation ($\omega$), comportant un dispositif de capteur de pression (220) et comportant un dispositif de détermination (110) pour la détermination de la viscosité ($\eta$) du sang dans le corps du patient,
   ledit dispositif de détermination comportant une canule (200) munie d'une pointe et implantable dans le corps d'un patient, la canule (200) présentant une interface de collecte (225) à travers laquelle le sang du patient peut s'écouler à l'intérieur de la canule (200) et une interface d'évacuation (230) opposée à l'interface de collecte (225) et à travers laquelle le sang peut sortir de l'intérieur de la canule (200), la roue à aubes (205) servant à transporter le sang de l'interface de collecte (225) vers l'interface d'évacuation (230) de la canule (200), et ledit dispositif de détermination comportant un capteur de débit volumique (215) disposé dans la pointe de la canule (200) et conçu comme un capteur à ultrasons Doppler pour la détection d'un débit volumique (Q) du sang du patient à travers la canule (200), le dispositif de capteur de pression (220) étant configuré pour la détection d'une différence de pression ($\Delta$p) de la pression du sang dans la zone de l'interface de collecte (225) et de la pression du sang dans la zone de l'interface d'évacuation (230), et le dispositif de détermination (110) étant configuré pour déterminer la viscosité ($\eta$) du sang dans le corps du patient à l'aide au moins du débit volumique (Q) détecté du sang et de la différence de pression ($\Delta$p) détectée du sang et de la vitesse de rotation ($\omega$) de la roue à aubes (205) ou alternativement pour ce faire à l'aide au moins du débit volumique (Q) détecté du sang et de la différence de pression ($\Delta$p) détectée du sang et d'une consommation électrique $P_{el}$ du dispositif d'entraînement (210) pour la roue à aubes (205).

2. Système d'assistance cardiaque selon la revendication 1, **caractérisé en ce que** le dispositif de détermination (110) est configuré pour déterminer la viscosité ($\eta$) à l'aide d'une relation fonctionnelle entre le débit volumique (Q) et la différence de pression ($\Delta$p) par rapport à la viscosité ($\eta$) et/ou à l'aide d'une table de consultation, en particulier une relation entre le débit volumique (Q) et la différence de pression ($\Delta$p) par rapport à la viscosité ($\eta$) étant stockée dans la table de consultation.

3. Système d'assistance cardiaque selon la revendication 1 ou 2, **caractérisé en ce que** la roue à aubes (205) est disposée au niveau de ou dans la zone de l'interface d'évacuation (230).

4. Système d'assistance cardiaque (100) selon l'une des revendications 1 à 3, **caractérisé en ce que** le dispositif de capteur de pression (220) présente au moins un capteur de pression différentielle et/ou deux capteurs de pression barométrique (235).

5. Système d'assistance cardiaque selon l'une des revendications 1 à 4, **caractérisé en ce que** le dispositif de capteur de pression (220) est configuré pour détecter une différence de pression ($\Delta$p) entre deux points de capteur et pour la fournir ou l'envoyer au dispositif de détermination (110).

6. Procédé (400) permettant de déterminer une viscosité ($\eta$) du sang d'un patient dans un système d'assistance cardiaque comportant une roue à aubes (205), comportant un dispositif d'entraînement (210) accouplé à la roue à aubes (205) et servant à entraîner la roue à aubes (205) à une vitesse de rotation ($\omega$),

ledit dispositif de détermination présentant une canule (200) implantable dans le corps d'un patient et qui présente une interface de collecte (225) par laquelle le sang du patient peut s'écouler à l'intérieur de la canule (200) ainsi qu'une interface d'évacuation (230) opposée à l'interface de collecte (225) et à travers laquelle le sang peut sortir de l'intérieur de la canule (200), la roue à aubes (205) servant à transporter le sang de l'interface de collecte (225) vers l'interface d'évacuation (230) de la canule (200),

la viscosité ($\eta$) du sang étant déterminée à l'aide au moins d'un débit volumique (Q) du sang détecté par un capteur de débit volumique (215) disposé dans une pointe de la canule (200) et conçu comme un capteur à ultrasons Doppler et d'une différence de pression ($\Delta p$) détectée de la pression du sang dans la zone de l'interface de collecte (225) et de la pression du sang dans la zone de l'interface d'évacuation (230) et de la vitesse de rotation ($\omega$) de la roue à aubes (205) pour le transport du fluide ; ou, alternativement pour ce faire, à l'aide au moins d'un débit volumique (Q) du sang détecté dans la zone de l'interface de collecte (225) dans la canule (200) au moyen d'un capteur de débit volumique (215) disposé dans une pointe de la canule et conçu comme un capteur à ultrasons Doppler et d'une différence de pression ($\Delta p$) détectée du sang dans la zone de l'interface de collecte (225) et dans la zone de l'interface d'évacuation (230) et d'une consommation électrique $P_{el}$ du dispositif d'entraînement (210) pour la roue à aubes (205).

# Fig. 1

Fig. 2

# Fig. 3

# Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2175770 B1
- US 7591777 B2
- EP 3088016 A1